(19) **Europäisches Patentamt / European Patent Office / Office européen des brevets**

(11) **EP 4 717 156 A1**

(12) **EUROPEAN PATENT APPLICATION**

(43) Date of publication:
01.04.2026 Bulletin 2026/14

(21) Application number: 25204430.0

(22) Date of filing: 24.09.2025

(51) International Patent Classification (IPC):
$A61B\ 5/00^{(2006.01)}$     $A61B\ 5/055^{(2006.01)}$
$A61B\ 5/11^{(2006.01)}$     $G01R\ 33/565^{(2006.01)}$
$G01R\ 33/567^{(2006.01)}$     $A61B\ 6/00^{(2024.01)}$

(52) Cooperative Patent Classification (CPC):
A61B 5/0077; A61B 5/055; A61B 5/1128;
A61B 5/721; G01R 33/283; G01R 33/56509;
G01R 33/5673; A61B 6/527

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 27.09.2024 JP 2024168489

(71) Applicant: **FUJIFILM Corporation**
**Tokyo 106-8620 (JP)**

(72) Inventor: **HIROKI, Shoji**
**Tokyo (JP)**

(74) Representative: **Dehns Germany Partnerschaft mbB**
**Theresienstraße 6-8**
**80333 München (DE)**

(54) **MEDICAL IMAGING APPARATUS AND BODY MOVEMENT DATA PROCESSING METHOD**

(57) Provided is a medical imaging apparatus (1) having a body movement process function, which can set a threshold value for determining a body movement in consideration of a body movement situation that varies depending on a subject or an examination part of the subject, and thereby effectively reduce an influence of the body movement on the subject in a medical image.

A medical imaging apparatus according to an aspect of the present invention comprises a unit that sets a threshold value for determining a body movement for each subject. An optically captured image of a subject acquired by an optical imaging device different from a medical imaging apparatus is used not only for detecting a body movement of the subject but also for determining a threshold value for determining the body movement, and the threshold value is used to determine the body movement.

**FIG. 5**

```
        START
          │
  ACQUIRE CAMERA IMAGE          ~ S1
          │
  CALCULATE DISTORTION          ~ S2
  CORRECTION AMOUNT
          │
  SET THRESHOLD VALUE           ~ S3
          │
  DETERMINE BODY MOVEMENT       ~ S4
          │
  DETERMINE PROCESS             ~ S5
          │
  GENERATE IMAGE                ~ S6
  AND DISPLAY IMAGE
          │
         END
```

EP 4 717 156 A1

Description

CROSS-REFERENCE TO RELATED APPLICATIONS

The present application claims priority under 35 U.S.C. § 119 to Japanese Patent Application No. 2024-168489, filed September 27, 2024. Each of the above application(s) is hereby expressly incorporated by reference, in its entirety, into the present application.

BACKGROUND OF THE INVENTION

1. Field of the Invention

The present invention relates to a medical imaging apparatus that performs imaging by disposing a subject as an examination target in an imaging space, such as a magnetic resonance imaging apparatus (hereinafter, referred to as an MRI apparatus), a CT apparatus, or a PET apparatus, and particularly relates to a technology for reducing an influence of measurement data affected by a body movement that has occurred in the subject during the imaging on an image in the medical imaging apparatus.

2. Description of the Related Art

A medical imaging apparatus can non-invasively grasp an internal tissue of an examination target, and is widely used in medical sites. One of the issues in obtaining a high-quality image with the medical imaging apparatus is a body movement of a subject during the imaging. The body movement of the subject includes a movement associated with breathing and a heartbeat and other unintended movements, for example, a movement associated with physiological reactions such as convulsions, sneezing, and coughing, and other sudden movements. Due to such a body movement, an artifact occurs in the image, and image quality deterioration caused by the artifact hinders an image diagnosis.
Among the body movements, breathing movements and pulsations are easily predicted from their periodicity, and various measures are taken, for example, imaging is performed at a time when the movement is smallest, and acquired measurement data is corrected based on the periodicity (for example, JP2023-027608A). The technology disclosed in JP2023-027608A discloses that a device that optically images the subject, such as an optical camera, may be used separately from the medical imaging apparatus, to acquire breath-holding information of a subject based on a moving image, which is captured by the optical camera. In addition, JP2023-027608A also discloses that distortion caused by a lens for wide angle-of-view imaging may be corrected for a camera image acquired by using the lens for wide angle-of-view imaging.
Meanwhile, for a movement other than a periodic movement, in the MRI apparatus, a method has also been proposed in which the movement of the subject is detected, and the necessity of re-measurement is determined using the obtained movement information, or the image is reconstructed by excluding the measurement data affected by the body movement (body movement correction reconstruction) (see JP2023-022669A). In addition, a method has also been proposed in which a pressure sensor is placed on a bed on which the subject lies, a body movement is determined from the pressure sensor output, and, in a case in which the body movement is detected during the imaging, control of switching a data correction method to a data correction method that allows movement correction is performed to obtain an MR image in which the artifact caused by the body movement is suppressed (see JP2010-162332A).

SUMMARY OF THE INVENTION

A level (magnitude), an occurrence frequency, a duration, and the like of the body movement greatly differ depending on the subject (patient) and an examination part. For example, there are patients who have difficulty in undergoing an examination for a long time without moving depending on age or a pathological condition, and, even with the same patient movement, the influence of the body movement on the image differs greatly depending on whether a part in which the body movement has occurred is the same as or close to the examination part (imaging target) or is remote from the examination part (imaging target).
In the related art, as a threshold value in a case of determining the body movement, for example, a threshold value set by a user from an experience value is used, and an individual difference in the body movement is not considered. In addition, the technology disclosed in JP2010-162332A discloses that a threshold value for determining the body movement is different for each imaging part in a case of switching the imaging method, but the threshold value is a value that is arbitrarily set by the user and may not necessarily be an appropriate threshold value depending on the patient.
Further, in a case in which the body movement is detected by an optical imaging device such as a camera, an obtained video is a two-dimensional image, and includes distortion due to a lens, internal parameters, and the like of the optical imaging device, and insufficient detection or excessive detection of the body movement may occur depending on a position of the imaging part in an optically captured image.

JP2023-027608A describes that the distortion of the optically captured image is corrected in the determination of the breath holding period, but the issue of insufficient detection or excessive detection of the body movement for each patient cannot be solved by simply correcting the distortion of the two-dimensional image.

An object of the present invention is to provide a technology that enables setting of a threshold value in consideration of a body movement situation that varies depending on a subject and an examination part thereof, and thereby makes it possible to perform a more appropriate body movement process.

In order to achieve the above-described object, a medical imaging apparatus according to an aspect of the present invention comprises a unit that sets a threshold value for determining a body movement for each subject. In the aspect of the present invention, an optically captured image of the subject acquired by an optical imaging device different from the medical imaging apparatus is used not only for detecting the body movement of the subject but also for determining the threshold value for determining the body movement, and the threshold value is used to determine the body movement.

That is, the aspect of the present invention relates to a medical imaging apparatus comprising: a measurement unit that collects measurement data for generating an image of a subject who is undergoing an examination; and a processor that receives an optically captured image from an optical imaging device that optically images a region including an examination target part of the subject, and analyzes a body movement of the subject based on the optically captured image. The processor sets a threshold value for determining the body movement of the subject based on the optically captured image obtained by imaging the subject, and specifies body movement influence data affected by the body movement of the subject in the measurement data collected by the measurement unit, based on the set threshold value.

A body movement data processing method is a body movement data processing method of monitoring a body movement of a subject who is undergoing imaging using a medical imaging apparatus and specifying measurement data affected by the body movement of the subject as body movement influence data, the body movement data processing method comprising: receiving an optically captured image from an optical imaging device that optically images a region including an examination target part of the subject and setting a threshold value for determining the body movement of the subject based on the optically captured image; and specifying body movement influence data affected by the body movement of the subject in measurement data collected by the medical imaging apparatus, based on the set threshold value.

According to the aspects of the present invention, the optically captured image of the subject that is the imaging target is used not only for detecting the body movement but also for determining the threshold value for determining the body movement, an appropriate threshold value can be set for each subject that is the imaging target, and an appropriate body movement process, for example, selection of the body movement correction method, switching of the imaging method, and the like can be performed. In addition, it is possible to obtain an image in which the influence of the body movement is suppressed, by deleting excessive measurement data or reducing the remaining of measurement data that should be deleted originally.

## BRIEF DESCRIPTION OF THE DRAWINGS

FIG. 1 is a block diagram of a medical imaging apparatus to which the present invention is applied.
FIG. 2 is a diagram showing an MRI apparatus as an example of the medical imaging apparatus.
FIG. 3 is a functional block diagram of a processor (body movement processing unit).
FIG. 4 is a diagram showing a process in Embodiment 1.
FIG. 5 is a flowchart showing an example of a body movement correction process by the processor.
FIG. 6 is a diagram showing a process of Modification Example 2 of Embodiment 1.
FIG. 7 is a flowchart showing an example of a process in Embodiment 2.

## DESCRIPTION OF THE PREFERRED EMBODIMENTS

Hereinafter, embodiments of the present invention will be described with reference to the drawings.

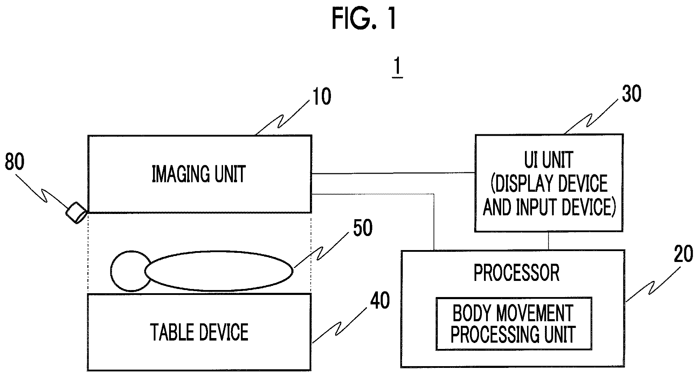

FIG. 1 shows configuration examples of a medical imaging apparatus according to the embodiment of the present invention and an optical imaging device used in the medical imaging apparatus. As shown in FIG. 1, the medical imaging apparatus 1 comprises a table device 40 or a gantry that provides an examination space for imaging a subject 50, a measurement unit (hereinafter, also referred to as an imaging unit) 10, and a processor 20. Further in the medical imaging apparatus 1 according to the present embodiment, one or a plurality of optical imaging devices 80 for monitoring the subject 50 disposed in the examination space, for example, monitoring cameras are disposed in the examination space or in the vicinity of the examination space. Although not shown, a device that measures biological signals such as a heartbeat and breathing of the subject 50, for example, an electrocardiograph or a pressure gauge may further be provided.

The configuration of the imaging unit 10 varies depending on the modality, and, for example, in a case of a CT apparatus, the imaging unit 10 includes a scanner equipped with an X-ray source and an X-ray detector, a high-voltage device that supplies a high voltage to the X-ray source, a drive source that drives the scanner, and the like. In addition, in a case of the MRI apparatus, the MRI apparatus includes a magnet that generates a static magnetic field in the examination space, a gradient magnetic field coil that generates a gradient magnetic field and a power supply thereof, a high-frequency generation device that generates a high-frequency magnetic field and a high-frequency transmission coil, and a high-frequency receive coil that receives a nuclear magnetic resonance signal.

The processor 20 has, for example, a function as a controller that controls the operation of the imaging unit 10 and a function as an operation unit that processes measurement data collected by the imaging unit 10 to generate or correct an image. Furthermore, the processor 20 according to the present embodiment processes an optically captured image from the optical imaging device 80 and the biological signal from a biological signal measurement device as necessary, to process the body movement of the subject 50.

The processor 20 can be configured by a known processing device, such as a computer comprising a CPU and a memory, a programmable IC, or a combination thereof. In a case of the computer, the process by the processor is realized by the CPU reading a program that implements each function of control or operation. All the functions of the processor can be implemented by a single processor, but each function can also be implemented by combining one or a plurality of processors. In FIG. 1, one processor 20 represents one or a plurality of processors, and shows individual functions implemented by one or a plurality of processors 20. In addition, a display device that displays an image or a GUI and an input device for a user to input a command, a necessary numerical value, or the like (collectively referred to as a UI unit 30) are connected to the processor 20, although not shown, the processor 20 can also have an interface function for communicating with or being connected to a cloud, a database, a storage device, or another image processing device outside the processor 20 in a wired manner.

Among the functions of the processor 20, the operations such as the control of the apparatus and the image generation are the same as those of a general medical imaging apparatus except for the functions related to the body movement process, and the description thereof will be omitted in the present specification. The content of the body movement process will be described in detail in the embodiment described below, but typically, the body movement of the subject 50 who is being imaged by the medical imaging apparatus 1 is acquired from the optically captured image, detection of a time point of occurrence of the body movement, magnitude of the body movement, and the like, specification of the measurement data (body movement influence data) affected by the body movement, process determination in accordance with the body movement influence data, and the like are performed. In the process determination, for example, it is determined whether or not it is necessary to re-capture the body movement influence data or whether or not the body movement influence data can be deleted to generate the image.

Here, in the body movement process in the present embodiment, before the body movement influence data is specified, an appropriate threshold value for determining the body movement of the subject who is undergoing the examination (in the middle of imaging by the medical imaging apparatus) is set using the optically captured image, and the determination of the body movement and the specification of the body movement influence data are performed using the threshold value. That is, a threshold value for determining the body movement is set for each target subject and further for each part of the subject. As a result, it is possible to appropriately determine the body movement in accordance with various forms of the body movement for each subject, to prevent the excessive detection or the insufficient detection of the body movement, and to efficiently perform the body movement correction with high accuracy.

Hereinafter, an embodiment of the process of the processor in the medical imaging apparatus according to the embodiment of the present invention will be described with a case in which the medical imaging apparatus is the MRI apparatus as an example. First, a configuration of the MRI apparatus and a flow of the process common to each embodiment will be described.

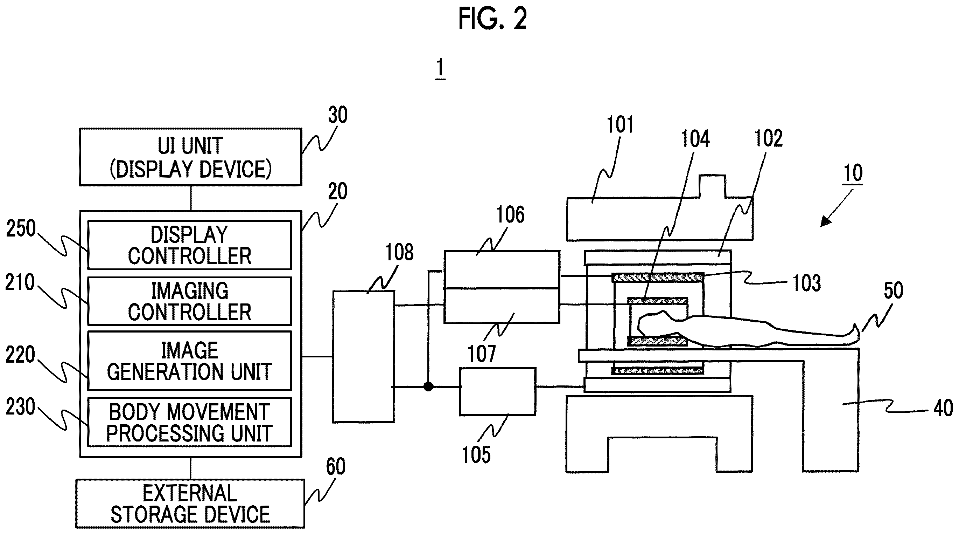

As shown in FIG. 2, the configuration of the imaging unit 10 is similar to that of a known MRI apparatus, and comprises a static magnetic field magnet 101 that generates a uniform magnetic field (static magnetic field) in the examination space in which a subject 50 is placed, a gradient magnetic field coil 102 that applies a gradient magnetic field to the static magnetic field, an RF transmission coil 103 that applies a high-frequency magnetic field to excite the nuclei of the atoms constituting the tissue of the subject, and an RF receive coil 104 that receives an NMR signal generated by the subject, in which the gradient magnetic field coil 102, the RF transmission coil 103, and the RF receive coil 104 are connected to a gradient magnetic field power supply 105, a transmitter 106, and a receiver 107, respectively.

The static magnetic field magnet 101, the gradient magnetic field coil 102, and the RF transmission coil 103 are housed in the gantry, and the subject 50 is positioned in the examination space in the gantry in a state of having the RF receive coil 104 attached to the examination part and being laid on the table device 40.

A sequencer 108 operates under the control of the processor 20, determines a pulse sequence for each scan using a pulse sequence type and imaging conditions such as imaging parameters set in advance or set by the user, operates the gradient magnetic field power supply 105, the transmitter 106, and the receiver 107 in accordance with the determined pulse sequence, and controls the imaging unit 10 to collect echo signals (k-space data) necessary for image reconstruction.

The processor 20 performs control of the imaging unit 10 via the sequencer 108, calculation such as image reconstruction using the k-space data, control of display of the UI unit 30, and processing of information input by the user. Since the functions and operations of the units in a case in which the imaging unit 10 collects the k-space data, as well as the process performed by the processor 20 related thereto, are the same as in a general MRI apparatus, detailed description thereof will be omitted here.

The optical imaging device 80 is disposed at one end portion of the gantry (entrance on a side into which the subject is inserted) and one or more places inside the gantry, images the subject before the imaging is started, and transmits the optically captured image to the processor 20. Hereinafter, as the optical imaging device 80, the monitoring camera, such as a monitoring camera comprising one or a plurality of cameras, a monitoring camera comprising a wide-angle lens or a fisheye lens, or an infrared camera that can continuously perform imaging by optical means to output time-series frame images (video) is used. In the following description, the optical imaging device 80 is also referred to as a monitoring camera, and the optically captured image is also referred to as a camera image or a frame image.

The processor 20 receives the frame image from the monitoring camera 80, detects the body movement between the frames, collates a timestamp of the frame image with an acquisition time of each echo data constituting the k-space data, and performs the body movement process by associating the passage of time of the collection of the k-space data with the body movement information.

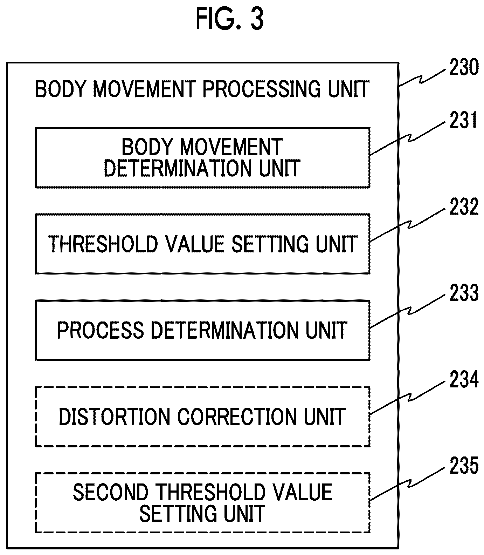

FIG. 3 is a block diagram showing an example of a function of the processor 20 related to the body movement process. As shown in FIG. 3, the body movement processing unit 230 comprises a body movement determination unit 231 that determines the magnitude and the duration of the body movement, a threshold value setting unit 232 that sets the threshold value for determining the body movement to be applied to the subject using the optically captured image of the subject, and a process determination unit 233 that determines a subsequent process based on the determination of the body movement determination unit 231. Further, as shown by a dotted line block, a distortion correction unit 234 that calculates the distortion of the camera image, a second threshold value setting unit 235 that sets a threshold value (second threshold value) related to the persistence of the body movement, and the like may be provided.

The processes of the units of the body movement processing unit 230 may be performed by a single processor or may be performed by a plurality of processors (including a computer, a programmable IC such as an ASIC or an FPGA).

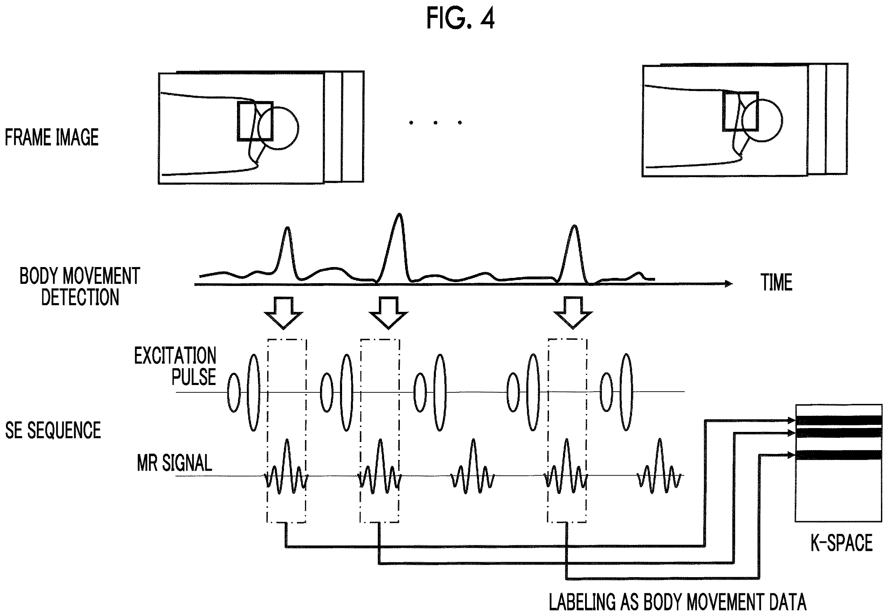

An outline of the body movement process of the processor 20 having the above-described configuration will be described with reference to FIG. 4.

The body movement processing unit 230 analyzes the frame image received from the monitoring camera 80, and the body movement determination unit determines the body movement that affects the image (body movement determination unit 231). In the analysis of the frame image, for example, an ROI is set in an image portion including a region of the subject to be monitored, a change in ROI between the frames is tracked by a method such as optical flow to obtain a movement vector, and the body movement is detected. In addition, in order to determine whether or not the body movement is the body movement that affects the image, a threshold value for determining the body movement is set for each subject (threshold value setting unit 232), and the determination is performed using the threshold value. The threshold value is set based on prior information, such as the tendency of the body movement of the subject which is the target, for example, whether a subject is likely to move frequently, a subject is likely to generate the body movement accompanied by a large spasm or the like, or a subject is likely to move a specific part such as limbs, or the information specified from the frame image.

Meanwhile, the body movement processing unit 230 acquires information on an imaging sequence (in FIG. 4, a spin echo (SE)-based sequence is shown) being executed by the imaging unit 10, and specifies the body movement influence data in the k-space data collected by the imaging sequence from the detected body movement and the imaging sequence in progress. Further, the body movement processing unit 230 determines the process such as re-capturing of the body movement influence data or performing image reconstruction in which the body movement influence data is corrected (body movement correction reconstruction) based on the position of the body movement influence data in the k-space, the number of body movement influence data, a proportion occupied by the body movement influence data in the k-space, and the like (process determination unit 233). For this process determination, a determination criterion, the number of body movement influence data, or a threshold value of the proportion occupied by the body movement influence data in the k-space is set in advance. Examples of the determination criterion include that the re-capturing is performed in a case in which the body movement influence data is present in a low-frequency range of the k-space, and that, in a case in which the body movement influence data is present only in a high-frequency range of the k-space, the re-capturing is performed in a case in which the number or the proportion of the body movement influence data in the k-space exceeds a predetermined threshold value, and the body movement correction reconstruction is performed in a case in which the number or the proportion of the body movement influence data in the k-space is equal to or less than the predetermined threshold value.

According to the present embodiment, in the body movement process of determining the body movement and determining the subsequent process in accordance with the k-space position, the number, and the like of the body movement influence data, it is possible to perform the process in accordance with the body movement characteristics of the subject by setting the threshold value for determining the body movement for each subject or each part. As a result, it is possible to prevent the insufficient detection or the excessive detection of the body movement, to prevent the extension of the imaging time due to insufficient body movement correction or unnecessary data re-capturing, and it is possible to efficiently obtain an image in which the influence of the body movement is suppressed.

Hereinafter, embodiments of the body movement determination and a specific method of setting the threshold value of the body movement determination will be described.

Embodiment 1

The present embodiment is characterized in that the processor 20 has a function (distortion correction unit 234) of correcting the distortion of the frame image acquired from the optical imaging device 80, and performs the setting of the threshold value and the detection of the body movement using the frame image after the distortion correction.

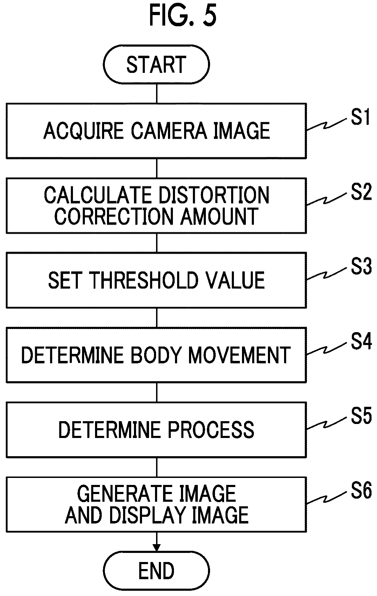

Hereinafter, the process in the present embodiment will be described with reference to FIG. 5. In the following description, the term "imaging" includes not only imaging (main imaging) for obtaining a target diagnosis image but also pre-measurement for determining conditions for the main imaging and imaging in other preparation stages.

In a case in which the subject is positioned in the imaging space at the start of the imaging, the monitoring camera 80 is operated before and after the subject is positioned in the imaging space, and the frame image of the monitoring camera 80 is acquired (S1). Next, the distortion correction unit 234 calculates the distortion (distortion parameter) of the camera image, and corrects the distortion of the frame image using the distortion parameter (S2).

In general, the main distortion that occurs in the camera image is (1) radial distortion and (2) tangential distortion, and the radial distortion is image distortion that increases as a distance from the center of the image increases, and can be represented by Expression (1).

$$x_{corrected} = x(1 + k_1 r^2 + k_2 r^4 + k_3 r^6)$$

$$y_{corrected} = y(1 + k_1 r^2 + k_2 r^4 + k_3 r^6) \qquad \text{Expression (1)}$$

In the expression, x and y represent positions (coordinates) on the image, r represents a distance from an origin of a focal point of the camera, and k1, k2, and k3 are coefficients.

The tangential distortion is distortion that occurs since the lens and the image plane are not perfectly parallel to each other, and this distortion causes a certain region to appear closer than expected. The tangential distortion can be represented by Expression (2).

$$x_{corrected} = x + [2p_1 xy + p_2(r^2 + 2x^2)]$$

$$y_{corrected} = y + [p_1(r^2 + 2y^2) + 2p_2 xy] \qquad \text{Expression (2)}$$

In the expression, p1 and p2 are coefficients. Each coefficient of Expression (1) and Expression (2) can be obtained by a method described later, and the distortion correction unit 234 stores the obtained coefficients as distortion parameters (distortion coefficients) of Expression (3).

$$Distortion\ coefficients = (k_1\ k_2\ p_1\ p_2\ k_3) \qquad \text{Expression (3)}$$

Further, as parameters that cause the distortion in the camera image, in addition to the above-described distortion parameters, there are internal parameters (Expression 4) called a camera matrix and external parameters (Expression 5) that are a matrix for converting world coordinates into camera coordinates. The internal parameters are values unique to the camera (known values).

$$camera\ matrix = \begin{bmatrix} f_x & 0 & c_x \\ 0 & f_y & c_y \\ 0 & 0 & 1 \end{bmatrix}$$

Expression (4)

In the expression, fx and fy are focal lengths (fx, fy), and cx and cy are optical centers (cx, cy) of the camera.

Camera coordinates External parameters World coordinates

$$\begin{bmatrix} X_c \\ Y_c \\ Z_c \end{bmatrix} = \begin{bmatrix} r_{11} & r_{12} & r_{13} & t_1 \\ r_{21} & r_{22} & r_{23} & t_2 \\ r_{31} & r_{32} & r_{33} & t_3 \end{bmatrix} \begin{bmatrix} X_w \\ Y_w \\ Z_w \\ 1 \end{bmatrix}$$

Expression (5)

The distortion of the camera image, that is, the above-described parameter for determining the distortion varies depending on the type of the monitoring camera and the installation location, and for example, in a case of a fisheye lens camera installed on a ceiling of the gantry, a center portion of the image is largely reflected and the distortion is small, but the distortion increases toward a peripheral portion and the image is reflected small. In the wide-angle lens camera, the distortion is larger toward the outside, but the image is larger on the outside than the center.

The distortion correction unit 234 estimates the distortion parameter and the external parameter by imaging by imaging, as the subject, a test chart composed of a pattern whose undistorted appearance is known, for example, a chessboard. That is, a shift amount from an original position of the test pattern is acquired from the camera image obtained by imaging the test chart, and the distortion parameter is estimated based on the shift amount.

The imaging for acquiring the distortion parameter can be performed at the time of development of the MRI apparatus, at the time of installation in a facility, and the like. For example, the imaging for acquiring the distortion parameter may be performed by installing a wide-angle camera on the ceiling directly above the table of the MRI apparatus in the MRI imaging room for experiment during the development of the apparatus, and in order to calculate the distortion parameter with higher accuracy, the service man may perform the imaging for acquiring the distortion parameter using the test chart described above at the time of installation of the MRI apparatus, at the time of regular maintenance, or the like for each hospital in which the MRI apparatus is installed. Accordingly, various variations such as actual installation height and installation position of the camera, and a lens variation of the camera can be eliminated.

Various known methods have been proposed for the calculation of the distortion parameter estimated as described above, an algorithm for distortion correction, or a simple distortion correction algorithm that does not use the distortion parameter (for example, see JP2013-258679A, JP2019-29978A, and the like for the wide-angle lens image, the distortion correction camera calibration, MathWorks camera calibration, and the wide-angle lens camera, and see JP2008-048443A, JP2008-061260A, and the like for the fisheye lens camera), and correction can be performed using these known methods.

By the distortion correction, an error due to the distortion of the movement amount due to the body movement between the center portion of the image and the edge portion of the image can be improved. That is, the movement amount due to the body movement at the center portion of the image and the movement amount due to the body movement at the edge portion of the image can be detected on substantially the same scale, and the threshold value of the body movement detection can be unified at the center portion and the edge portion.

The distortion correction unit 234 applies the distortion parameter to the frame image used for the subsequent body movement determination, to perform distortion correction. In a case in which an ROI for specifying a region (an examination part, a vicinity of the examination part, or the like) in which the body movement is to be monitored is set in the frame image, the distortion correction may be performed on the ROI.

The threshold value setting unit 232 sets the threshold value of the body movement based on the frame image after the distortion correction (S3). The threshold value of the body movement is determined, for example, in the following manner. First, various amounts (statistics) such as a body movement level of the examination part of the subject, a range of a body movement amount during a predetermined period, a maximum value, a minimum value, an average value, and a deviation are calculated based on a plurality of frame images during a rest period in which a sudden body movement other than the periodic body movement associated with a life activity such as breathing movement or heartbeat does not occur. The threshold value of the body movement detection is determined based on this various amounts.

For example, a maximum value or an average value, a deviation ($\sigma$), a value obtained by multiplying these various amounts by a predetermined coefficient, or a value calculated by using a function of these various amounts or coefficients (for example, average value + nσ or average value - nσ) is used as the threshold value. Here, since the body movement amount varies depending on whether the examination part is the head, the chest, the abdomen, or the limbs due to the influence of breathing or heartbeat even in a rest state, the threshold value or the predetermined coefficient is determined in consideration of this. For example, the threshold value is set to be small in the head on which the influence of the periodic movement is relatively small, and the threshold value is set to be large in a part on which the influence of the periodic movement is large. In this case, the influence of the periodic movement can be corrected by another method. In addition, the predetermined coefficient may be set by the user or may be set on the apparatus side depending on whether the influence of the body movement is severely treated or leniently treated in accordance with the image quality to be obtained. Further, the threshold value may be adjusted in consideration of the prior information obtained regarding the tendency of the body movement of the subject.

In this way, the coefficient to be multiplied by the statistics of the body movement obtained in a rest state is made different depending on the part in which the tendency of the body movement is different, and the threshold value is set for each part. As a result, appropriate body movement determination can be performed using the threshold value.

Then, the body movement determination unit 231 performs the detection and determination of the body movement, that is, the determination of whether or not the body movement has a large influence on the image, on the frame image after the distortion correction using the set threshold value (S4). That is, in a case in which the level of the body movement exceeds the set threshold value, it is determined that the body movement has occurred. In a case in which the threshold value is set for each part, the determination is performed for each part to determine the body movement for each part. The determination may be performed before the start of the imaging. Then, in a case in which the imaging in accordance with the imaging sequence is started by the imaging unit 10, as shown in FIG. 4, the progress of the imaging sequence (for example, the main imaging) and the time at which it is determined that the body movement has occurred are associated with each other, and the measurement data (body movement influence data) collected at the time is labeled (specified).

The process after the body movement influence data is specified is the same as, for example, the process described in JP2023-022669A and the like, and is determined in accordance with the number of body movement influence data and the position of the body movement influence data in the k-space (S5). As an example, in a case in which the body movement influence data is present in a low-frequency region of the k-space, the imaging controller 210 performs re-capturing (re-measurement) of the data under the control of the imaging controller 210. In a case in which the body movement influence data is present only in the high-frequency region of the k-space, it is determined whether or not to perform the re-measurement in accordance with the number or the position of the body movement influence data. For example, in a sequence of sequential ordering in which a scan is performed from one end to the other end along a phase-encoding direction of the k-space, in a case in which the k-space data from 0 encoding to one end portion can be collected and the body movement occurs in a case of moving toward the other end portion, body movement correction reconstruction is performed using a method of data estimation or the like without re-capturing. In a case in which the number of specified body movement influence data is large or the range of the body movement influence data is large in a case of scanning from one end portion of the k-space toward the center, the process is determined based on the determination criterion such as performing re-capturing.

Finally, in a case in which the collection of the k-space data that can be subjected to image reconstruction is completed, a image generation unit 220 performs image reconstruction (including body movement correction reconstruction) to generate the image. The generated image is displayed on the display device of the UI unit 30 as a display image by the display controller 250 together with accessory information such as information on the subject and the imaging conditions (S6).

In addition to the subject image which is the final image, the display device may display, for example, a threshold value (a numerical value or a qualitative display indicating whether the value is a strict value or a gentle value with respect to the image quality) for the subject, which is a condition for the body movement correction, and/or k-space data in which the body movement determination situation and the body movement influence data are specified as shown in FIG. 4, and these may be performed in the middle of the progress of the steps described above.

According to the present embodiment, it is possible to set the threshold value and optimize the body movement determination using the threshold value by correcting the distortion of the frame image caused by the configuration of the monitoring camera 80 on the premise that the threshold value for determining the body movement is determined by analyzing the frame image from the monitoring camera (optical imaging device) 80 and the body movement determination is performed. In addition, by setting the threshold value using the camera image of the subject in a rest state, it is possible to perform the body movement determination with high accuracy for the frame images of various subject states acquired thereafter.

Modification Example 1 of Embodiment 1

In Embodiment 1, the distortion correction unit 234 corrects the distortion of the frame image using the distortion correction amount calculated by the distortion correction unit 234, but it is also possible to set the threshold value in consideration of the distortion without correcting the distortion. In the present modification example, the process of the distortion correction unit 234 is replaced with the process of only distortion calculation.

In the present modification example, for example, the distortion correction unit 234 calculates the parameters such as the distortion parameter described above for the monitoring camera mounted on the medical imaging apparatus. The method of calculating the parameters is the same as the method in Embodiment 1.

Next, the threshold value setting unit 232 calculates various amounts of information, such as the body movement level of the examination part of the subject, the range of the body movement amount for the predetermined period, the maximum value, the minimum value, the average value, and the deviation, using only the center region of the plurality of frame images, and the threshold value setting unit sets the threshold value using the various amounts of the body movement information calculated for the center portion of the image. Here, the movement vector (movement amount due to the body movement) value between the frames obtained for the camera image that is not subjected to the distortion correction is different between the center portion of the image and the edge portion of the image. Therefore, for portions other than the center portion of the image, the threshold value is adjusted using the distortion parameter calculated by the distortion correction unit 234, and for example, the threshold value is adjusted such that the threshold value increases toward the edge of the image in which the distortion is large. That is, a different threshold value depending on the region of the image is set.

As a result, the threshold value itself is a value reflecting the distortion, so that it is possible to perform the body movement determination reflecting the distortion.

Modification Example 2 of Embodiment 1

Although, in Embodiment 1, the threshold value is set for the movement vector (body movement amount) on the image using the frame image which is a planar image, and the body movement determination is performed, in the present modification example, a three-dimensional position is detected using a plurality of camera images, and the body movement determination is performed with respect to a fluctuation in three-dimensional position.

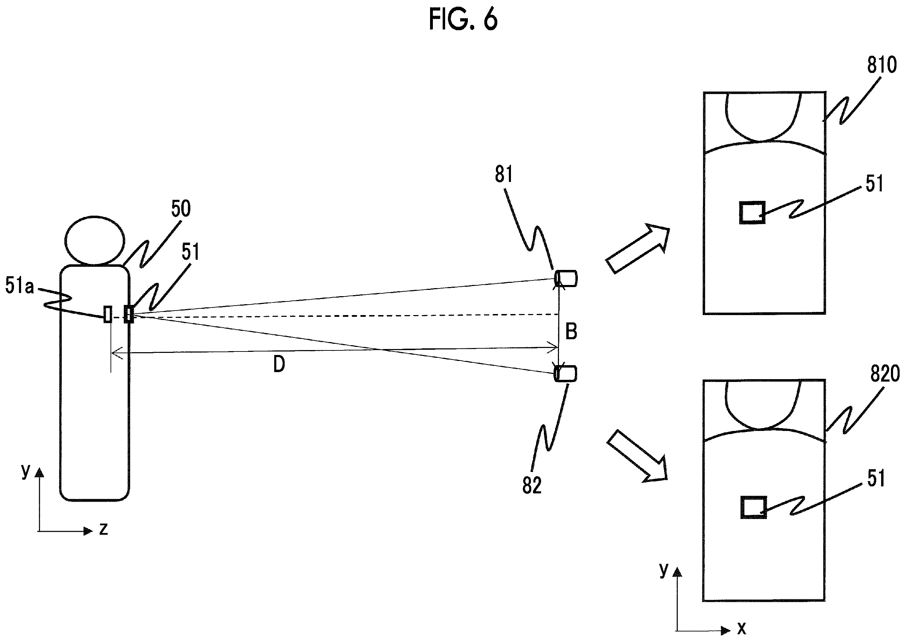

Hereinafter, an embodiment in which the three-dimensional position is corrected using camera images 810 and 820 acquired by two monitoring cameras 81 and 82 will be described with reference to FIG. 6.

As shown in FIG. 6, in the camera images obtained by imaging a predetermined region or a feature point 51 of the subject 50 with the two monitoring cameras 81 and 82, in a case in which an image plane is defined as an x-y plane, the fluctuation in position of the feature point 51 in the plane, that is, a change in position of the feature point 51 in the x direction and the y direction can be seen, but, for example, even in a case in which the feature point 51 is changed to a position of a feature point 51a, it is not possible to grasp the change in position in the z direction (depth direction). Meanwhile, the positions of the feature point 51 on the two camera images 810 and 820 are different from each other due to the difference in camera position. The feature point 51 on the image is shifted in the y direction in a case in which the two monitoring cameras 81 and 82 are disposed to be spaced apart from each other in the y direction, and the feature point 51 on the image is shifted in the x direction in a case in which the two monitoring cameras 81 and 82 are disposed to be spaced apart from each other in the x direction. In a case in which the shift amount of the positions (two coordinates) of the feature point 51 in the images of the two monitoring cameras 81 and 82 is known, the position of the feature point 51 in the z direction can be calculated by using the shift amount. That is, in a case in which the shift amount of the positions of the feature point 51 is denoted by S, a focal length of the camera is denoted by f, and a distance between the focal points of the two cameras is denoted by B, a distance D (distance in the z direction) from the focal point to the feature point 51a can be calculated by the following Expression.

$$D = B \times f/S$$

The shift amount S in the camera images of the two monitoring cameras 81 and 82 can be calculated by, for example, a method such as block matching. For example, one image is divided into a plurality of regions as a reference, a region having a high correlation in the other image is determined for each region, and the shift amount S between the images is calculated for each frame. As a result, the fluctuation in distance D for each region can be obtained.

Since the fluctuation in three-dimensional position of the predetermined region (ROI) or the feature point can be obtained from the positions and the focal lengths of the two monitoring cameras and the shift amount of the camera images in this way, the fluctuation, that is, the body movement is determined using the threshold value of the body movement. In this case, the threshold value is also determined based on the fluctuation in three-dimensional position.

As the method of detecting the three-dimensional position from the plurality of camera images, in addition to the above-described method, there is a known method (for example, JP2022-094744A), and the method is not limited to the above-described method, and other known methods can also be used.

The method of setting the threshold value after the body movement detection and determining the body movement is the same as the method in Embodiment 1, and the description thereof will be omitted. In addition, in the present modification example, the necessary distortion correction may be performed or the threshold value considering the distortion may be set in accordance with the lens characteristic of the monitoring camera, and the body movement determination with higher accuracy can be performed.

Embodiment 2

In the present embodiment, the body movement detection in the embodiment 1 is executed in a pre-scan (pre-measurement), and the result of the pre-scan is reflected in the determination of the imaging method or the change in imaging conditions of the main imaging. In a case of an MRI apparatus, the pre-scan includes measurement or imaging performed by disposing the subject in the imaging space, such as pre-measurement for correction of a static magnetic field inhomogeneity or the like performed before the main imaging for obtaining the diagnosis image of the subject, and imaging for positioning the subject at a desired imaging position or determining an imaging cross section, and may be any of the measurement or the imaging.

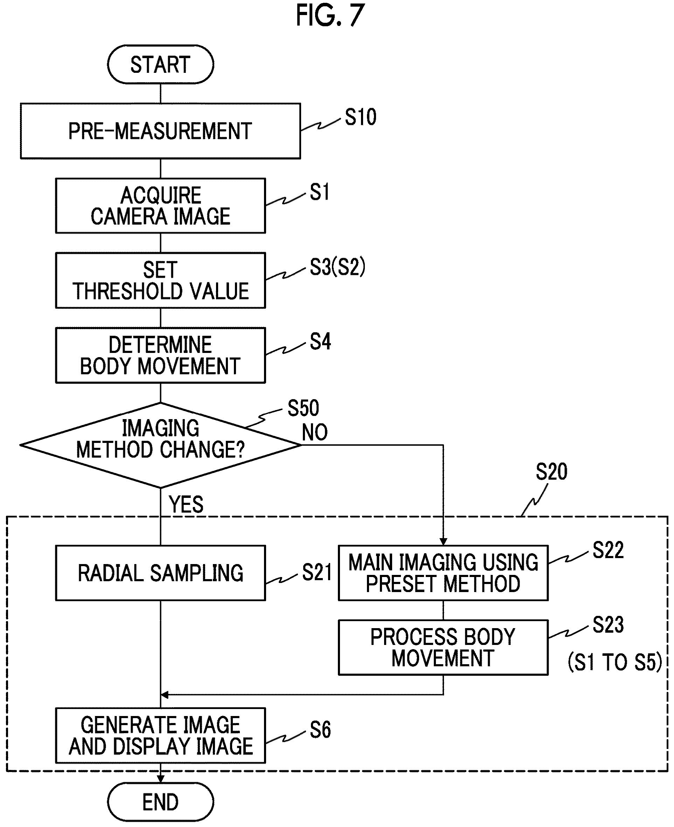

A flow of the process in the present embodiment will be described with reference to a flowchart of FIG. 7. In FIG. 7, the process having the same content as the process of FIG. 5 is denoted by the same reference numerals, and the duplicated description will be omitted.

In a case in which the pre-scan is started before the main imaging (S10), the following operations are performed in a similar manner to those in Embodiment 1: before and after the start of the pre-scan, acquiring the camera image of the monitoring camera (S1), setting the threshold value of the predetermined part or setting the threshold value for each part (S3), preferably correcting the distortion of the camera image in such a case (S2), and determining the body movement using the threshold value (S4).

Meanwhile, the body movement processing unit 230 calculates a proportion of a time for which the body movement determination unit 231 determines that the body movement has occurred to the entire execution time of the pre-scan, and determines whether the proportion is within a predetermined threshold value (a threshold value of the body movement time) or exceeds the threshold value. The predetermined threshold value may be determined in advance as a numerical value of 30%, 50%, or the like, or may be set by the user in accordance with the subject or the imaging purpose. For example, in some cases, it may be difficult for the subject to remain stationary due to a disease. For such a patient, a somewhat higher threshold value may be set, such as 50%. In addition, the threshold value can be set to be high even in a case in which the data collection method that is set in advance is a data collection method that is less likely to be affected by the body movement to some extent. In order to distinguish the two threshold values, the threshold value of the body movement level set in the process of S3 is referred to as a first threshold value, and the threshold value of the occurrence period of the body movement is referred to as a second threshold value.

The body movement processing unit 230 determines whether or not to change the imaging method of the main imaging by using these first and second threshold values. Hereinafter, as an example, a case will be described in which the data collection method is changed. For example, in a case in which the occurrence time of the body movement during which the body movement exceeds the first threshold value (in a case in which a plurality of times of the body movement have occurred, a total time thereof may be used) exceeds the second threshold value at the end of the pre-scan, the data collection method in the main imaging is changed to the data collection method that is less likely to be affected by the body movement (S50).

Examples of the data collection method that is less likely to be affected by the body movement include echo planar imaging (EPI) that can collect k-space data (phase encoding data) to be collected after one excitation, radial scan that collects data radially with respect to an origin of the k-space, and a method (referred to as a propeller scan) of collecting data while rotating a parallel data block (blade) including phase encoding radially. Any of these data collection methods robust to the body movement may be set in advance, or a data collection method that can be changed may be presented to the user through the UI unit 30 such that the user can select the data collection method.

In a case in which the body movement during the pre-scan is equal to or less than the second threshold value, the method set in advance as the data collection method in the main imaging is employed as it is, and the main imaging is performed in the same manner as in Embodiment 1 (S22, S23). That is, the frame image sent from the monitoring camera during imaging is monitored, the body movement is detected, the body movement influence data is specified, and the process determination is performed, such as determining whether performing the measurement of the unmeasured data including the body movement influence data or performing the body movement correction in accordance with the position of the specified body movement influence data in the k-space or the number of data (S23).

In a case in which the k-space data that can be subjected to image reconstruction is finally collected regardless of whether the data collection method is changed or not changed, the image is reconstructed (including the body movement correction reconstruction), and the image is displayed or the image data is stored (S6).

In a case in which the data collection method set in advance is a pulse sequence that is robust to the body movement, the main imaging may be performed with the set data collection method as it is, or for example, among the set imaging parameters, a parameter that can further shorten the time, for example, a parallel imaging (PI) speed-up factor, a repetition time TR, a slice thickness, the number of additions, or the like may be changed. Such a change is also included in the "change in imaging method" according to the present embodiment. For such a change in imaging parameter, options may be presented to the user through the UI unit 30, and the designation of the user may be received, or the setting may be performed on the apparatus side in accordance with a predetermined priority, and the result may be presented to the user.

According to the present embodiment, in the pre-scan executed before the main imaging, the body movement information including the tendency of the body movement is acquired, and the imaging method in the main imaging is set based on the body movement information, so that it is possible to acquire an image in which the influence of the body movement is suppressed while the time required for the main imaging is minimized.

Although the embodiments of the present invention has been described above using the MRI apparatus as an example, the present invention can be applied to a medical imaging apparatus having a function of receiving information from an optical imaging device such as a monitoring camera. In addition, the present invention is not limited to the embodiments or modification examples described above, appropriate combinations thereof, omission of components or processes that are not essential for implementing the present invention, and addition of known components or processes are all included within the scope of the present invention.

Explanation of References

10: imaging unit
20: processor
30: UI unit
40: table device
50: subject
80, 81, 82: monitoring camera (optical imaging device)
230: body movement processing unit

**Claims**

1. A medical imaging apparatus (1) comprising:

    a measurement unit (10) that collects measurement data for generating an image of a subject (50) who is undergoing an examination; and
    a processor (20) that receives an optically captured image from an optical imaging device that optically images a region including an examination target part of the subject, and analyzes a body movement of the subject based on the optically captured image,
    wherein the processor sets a threshold value for determining the body movement of the subject based on the optically captured image obtained by imaging the subject, and specifies body movement influence data affected by the body movement of the subject in the measurement data collected by the measurement unit, based on the set threshold value.

2. The medical imaging apparatus according to claim 1,
    wherein the processor (20) corrects distortion of the optically captured image, determines magnitude of the body movement using the corrected optically captured image, and specifies the body movement influence data.

3. The medical imaging apparatus according to claim 1,
    wherein the processor (20) adjusts a threshold value used in a case in which the body movement influence data is specified, based on distortion of the optically captured image.

4. The medical imaging apparatus according to claim 1,
    wherein the processor (20) selects, as an image at a stationary state, an optically captured image obtained when a change in body movement is smallest from among a plurality of optically captured images obtained in time series by the optical imaging device, and calculates the threshold value using the image at the stationary state.

5. The medical imaging apparatus according to claim 1,
    wherein the processor (20) divides the optically captured image into a plurality of regions, and sets a threshold value used in a case in which the body movement influence data is specified, for each region.

6. The medical imaging apparatus according to claim 1,

wherein measurement performed by the measurement unit (10) includes main measurement for generating an image of the subject and pre-measurement performed prior to the main measurement, and the processor (20) analyzes a body movement of the subject who is undergoing the pre-measurement, and determines an imaging method of the main measurement based on a result of the analysis.

7. The medical imaging apparatus according to claim 6,

wherein the measurement unit (10) includes an imaging unit that is operated under predetermined imaging conditions, and collects a nuclear magnetic resonance signal generated from the subject, and the processor (20) changes the imaging conditions of the main measurement in a case in which a proportion or an occurrence frequency of the body movement influence data in the measurement data collected during the pre-measurement is equal to or higher than a predetermined threshold value.

8. The medical imaging apparatus according to claim 7,
wherein the imaging conditions include a pulse sequence and an imaging parameter used for imaging.

9. The medical imaging apparatus according to claim 1,
wherein the processor (20) acquires a plurality of optically captured images from a plurality of optical imaging devices disposed at at least two different positions, and detects the body movement using parallax between the plurality of optically captured images.

10. A body movement data processing method of monitoring a body movement of a subject (50) who is undergoing imaging using a medical imaging apparatus and specifying measurement data affected by the body movement of the subject as body movement influence data, the body movement data processing method comprising:

receiving an optically captured image from an optical imaging device that optically images a region including an examination target part of the subject and setting a threshold value for determining the body movement of the subject based on the optically captured image; and specifying body movement influence data affected by the body movement of the subject in measurement data collected by the medical imaging apparatus, based on the set threshold value.

11. The body movement data processing method according to claim 10,

wherein distortion of the optically captured image is corrected, and the body movement of the subject who is undergoing imaging is detected using the corrected optically captured image, and the body movement influence data is specified.

12. The body movement data processing method according to claim 10,

wherein distortion included in the optically captured image received from the optical imaging device is calculated, and a threshold value of the body movement used in a case in which the body movement influence data is specified is determined based on the distortion.

# FIG. 1

<u>1</u>

EP 4 717 156 A1

## FIG. 2

EP 4 717 156 A1

# FIG. 3

| BODY MOVEMENT PROCESSING UNIT | 230 |
| --- | --- |
| BODY MOVEMENT DETERMINATION UNIT | 231 |
| THRESHOLD VALUE SETTING UNIT | 232 |
| PROCESS DETERMINATION UNIT | 233 |
| DISTORTION CORRECTION UNIT | 234 |
| SECOND THRESHOLD VALUE SETTING UNIT | 235 |

# FIG. 4

FRAME IMAGE

BODY MOVEMENT
DETECTION

TIME

EXCITATION
PULSE

SE SEQUENCE

MR SIGNAL

K-SPACE

LABELING AS BODY MOVEMENT DATA

EP 4 717 156 A1

## FIG. 5

START

ACQUIRE CAMERA IMAGE — S1

CALCULATE DISTORTION CORRECTION AMOUNT — S2

SET THRESHOLD VALUE — S3

DETERMINE BODY MOVEMENT — S4

DETERMINE PROCESS — S5

GENERATE IMAGE AND DISPLAY IMAGE — S6

END

# FIG. 6

EP 4 717 156 A1

# FIG. 7

```
                    ┌──────────┐
                    │  START   │
                    └────┬─────┘
                         │
         ┌───────────────────────────┐
         │     PRE-MEASUREMENT        │⟿ S10
         └───────────────┬───────────┘
                         │
         ┌───────────────────────────┐
         │        ACQUIRE             │⟿ S1
         │     CAMERA IMAGE           │
         └───────────────┬───────────┘
                         │
         ┌───────────────────────────┐
         │         SET                │⟿ S3(S2)
         │   THRESHOLD VALUE          │
         └───────────────┬───────────┘
                         │
         ┌───────────────────────────┐
         │      DETERMINE             │⟿ S4
         │    BODY MOVEMENT           │
         └───────────────┬───────────┘
                         │
                    ◇─────────────◇ S50
                   │   IMAGING     │  NO
                   │ METHOD CHANGE?│─────────┐
                    ◇─────────────◇          │
                         │ YES               │
                         │                   ▼
         ┌───────────┐       ┌─────────────────────┐
         │  RADIAL   │⟿ S21  │ MAIN IMAGING USING  │⟿ S22
         │ SAMPLING  │       │   PRESET METHOD     │
         └─────┬─────┘       └──────────┬──────────┘
               │             ┌─────────────────────┐
               │             │  PROCESS BODY       │⟿ S23
               │             │   MOVEMENT          │ (S1 TO S5)
               │             └──────────┬──────────┘
               │◄───────────────────────┘
               │
         ┌───────────────────────────┐
         │    GENERATE IMAGE          │⟿ S6
         │  AND DISPLAY IMAGE         │
         └───────────────┬───────────┘
                         │
                    ┌──────────┐
                    │   END    │
                    └──────────┘
```

S20

**EUROPEAN SEARCH REPORT**

Application Number

EP 25 20 4430

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/193990 A1 (KANEKO YUKIO [JP] ET AL) 13 June 2024 (2024-06-13) | 1,3,4, 10,12 | INV. A61B5/00 |
| Y | * paragraphs [0005] - [0017] *<br>* paragraphs [0032] - [0039] *<br>* paragraphs [0064] - [0073] *<br>* paragraph [0083] * | 2,5-9,11 | A61B5/055 A61B5/11 G01R33/565 G01R33/567 |
|  | ----- | | |
| X | US 2018/350081 A1 (HSIEH JIANG [US]) 6 December 2018 (2018-12-06)<br>* paragraph [0010] *<br>* paragraph [0017] *<br>* paragraphs [0034] - [0038] *<br>* paragraph [0060] * | 1,4,5,10 | ADD. A61B6/00 |
|  | ----- | | |
| Y | US 2023/054048 A1 (OZAWA SHINYA [JP] ET AL) 23 February 2023 (2023-02-23)<br>* paragraph [0041] * | 2,11 | |
|  | ----- | | |
| Y | US 2010/152568 A1 (KOKUBUN KATSUTOSHI [JP]) 17 June 2010 (2010-06-17)<br>* paragraph [0069] *<br>* paragraphs [0098] - [0099] * | 5 | |
|  | ----- | | TECHNICAL FIELDS SEARCHED (IPC) |
| Y | US 2023/045497 A1 (SHOJI HIROKI [JP] ET AL) 9 February 2023 (2023-02-09)<br>* paragraphs [0026] - [0032] *<br>* paragraph [0066] *<br>* paragraphs [0084] - [0088] * | 6-8 | A61B G01R |
|  | ----- | | |
| Y | US 2022/183583 A1 (SASAKI TORU [JP]) 16 June 2022 (2022-06-16)<br>* claims 1-2 * | 9 | |
|  | ----- | | |
| X,P | EP 4 603 856 A1 (FUJI FILM CORP [JP]) 20 August 2025 (2025-08-20)<br>* paragraphs [0081] - [0086] * | 1,10 | |
|  | ----- | | |

The present search report has been drawn up for all claims

| Place of search | Date of completion of the search | Examiner |
|---|---|---|
| The Hague | 8 January 2026 | Loveniers, Kris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 4 717 156 A1

### ANNEX TO THE EUROPEAN SEARCH REPORT
### ON EUROPEAN PATENT APPLICATION NO.

EP 25 20 4430

This annex lists the patent family members relating to the patent documents cited in  the above-mentioned European search report.
The members are as contained in the European Patent Office EDP file on
The European Patent Office is in no way liable for these particulars which are merely given for the purpose of information.

08-01-2026

| Patent document cited in search report | | | Publication date | Patent family member(s) | | | Publication date |
|---|---|---|---|---|---|---|---|
| US | 2024193990 | A1 | 13-06-2024 | CN | 118141357 | A | 07-06-2024 |
| | | | | EP | 4382036 | A1 | 12-06-2024 |
| | | | | JP | 2024082179 | A | 19-06-2024 |
| | | | | US | 2024193990 | A1 | 13-06-2024 |
| US | 2018350081 | A1 | 06-12-2018 | CN | 108968996 | A | 11-12-2018 |
| | | | | DE | 102018112301 | A1 | 06-12-2018 |
| | | | | US | 2018350081 | A1 | 06-12-2018 |
| US | 2023054048 | A1 | 23-02-2023 | JP | 2023027608 | A | 02-03-2023 |
| | | | | US | 2023054048 | A1 | 23-02-2023 |
| US | 2010152568 | A1 | 17-06-2010 | CN | 101779957 | A | 21-07-2010 |
| | | | | JP | 5377219 | B2 | 25-12-2013 |
| | | | | JP | 2010162332 | A | 29-07-2010 |
| | | | | US | 2010152568 | A1 | 17-06-2010 |
| US | 2023045497 | A1 | 09-02-2023 | CN | 115919283 | A | 07-04-2023 |
| | | | | JP | 7461913 | B2 | 04-04-2024 |
| | | | | JP | 2023022669 | A | 15-02-2023 |
| | | | | US | 2023045497 | A1 | 09-02-2023 |
| | | | | US | 2024264258 | A1 | 08-08-2024 |
| US | 2022183583 | A1 | 16-06-2022 | JP | 7710838 | B2 | 22-07-2025 |
| | | | | JP | 2022094744 | A | 27-06-2022 |
| | | | | US | 2022183583 | A1 | 16-06-2022 |
| EP | 4603856 | A1 | 20-08-2025 | CN | 120477742 | A | 15-08-2025 |
| | | | | EP | 4603856 | A1 | 20-08-2025 |
| | | | | JP | 2025124553 | A | 26-08-2025 |
| | | | | US | 2025258261 | A1 | 14-08-2025 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2024168489 A
- JP 2023027608 A
- JP 2023022669 A
- JP 2010162332 A
- JP 2013258679 A

- JP 2019029978 A
- JP 2008048443 A
- JP 2008061260 A
- JP 2022094744 A